# EUROPEAN PATENT SPECIFICATION

(11) **EP 2 933 241 B1**
(45) Date of publication and mention of the grant of the patent: **14.03.2018**
(21) Application number: 13818238.1
(22) Date of filing: 12.12.2013
(51) Int. Cl.: C04B 38/00, A61L 27/12

(54) **METHOD FOR PRODUCING A POROUS CALCIUM POLYPHOSPHATE STRUCTURE**
VERFAHREN ZUR HERSTELLUNG EINER PORÖSEN CALCIUMPOLYPHOSPHATSTRUKTUR
PROCÉDÉ DE PRODUCTION D'UNE STRUCTURE POREUSE EN POLYPHOSPHATE CALCIQUE

(30) Priority: 12.12.2012 ES 201201225
(43) Date of publication of application: 21.10.2015
(73) Proprietor: Biotechnology Institute, I MAS D, S.L., 01005 Vitoria (ES)
(72) Inventor: ANITUA ALDECOA, Eduardo, E-01005 Vitoria - Alava (ES)
(74) Representative: Cueto, Sénida
(86) International application number: PCT/ES2013/000274
(87) International publication number: WO 2014/091036

(56) References cited:
- EP-A1- 1 449 818
- WO-A1-03/055418
- WO-A1-2012/117218
- WO-A2-2004/007399
- US-A1- 2010 173 009
- WEI J ET AL.: "Hierarchically microporous/macroporous scaffold of magnesium-calcium phosphate for bone tissue regeneration", BIOMATERIALS, vol. 31, 2010, pages 1260-1269, XP002723376, cited in the application
- WANG K.: "The effect of a polymeric chain-like structure on the degradation and cellular biocompatibility of calcium polyphosphate", MATERIALS SCIENCE AND ENGINEERING C, vol. 28, 2008, pages 1572-1578, XP002723377, cited in the application

## Description

### Technical field

The invention refers to a method for producing a porous calcium polyphosphate structure that serves as a biomaterial capable of being used for bone regeneration or other applications in different fields of medicine.

### Prior art

Bone regeneration methods are a common practice in orthopaedics, odontology and other fields of medicine for treating patients suffering from bone loss due to trauma, infections and tumours. Quite frequently, bone regeneration methods involve the use biomaterials for various purposes: to act as filling material; to act as a support for bone regeneration; and to encourage bone regeneration, among others. Biomaterials are characterised as being capable of interacting with the biological system of the patient, and in particular, of interfacing with biological systems for the purpose of evaluating, treating, increasing or replacing a tissue, organ or bodily function (Planell E, Gil M, Ginebra M. Biomaterials. In: Viladot V. Lecciones basicas de biomecanica del aparato locomotor (The Fundamentals of Locomotor Biomechanics). 1st Ed. Barcelona: Springer-Verlag Iberica: 2000. p. 291-304).

The most effective biomaterial in the field of bone regeneration is autologous bone, i.e. bone taken from the patient themselves. Autologous bone, however, is limited in terms of quantity and shape, and its extraction requires additional surgery, thereby increasing the risk of surgical complications. Alternatives to the use of autologous bone that are less traumatic for the patient have been developed over time and with the advance of technology. One of the most important and effective alternative to using autologous bone is the use of calcium phosphates, which are biocompatible, osteoconductive and absorbable. Among calcium phosphates, calcium orthophosphates have attracted much of the interest of the scientific community. Calcium polyphosphates (also known as calcium metaphosphates) are another biocompatible and absorbable alternative.

Usually, for a biomaterial to be able to provide an optimised catalisation of bone regeneration, the biomaterial has to be porous similarly to real bone tissue. Thus, the biomaterial must be capable of being converted or formed into a porous structure. In the case of calcium polyphosphate being used, its porous structures are generated by heat treating monocalcium phosphate (MCP), i.e. Ca(H₂PO₄)₂.H₂O or Ca(H₂PO₄). Prior art contains several known examples of methods of manufacturing porous calcium polyphosphate structures based on this treatment.

An exemplary method, described in Pilliar RM, et al. Biomaterials 2001;22:963-973, consists in heating the MCP at 500°C for ten hours and then melting it at 1100°C for one hour; colling the material very quickly in order to produce an amorphous compound; selecting particles presenting a granule size within a suitable range; finally, heating the selected particles at a temperature of 970°C for two hours.

In another example, patent application no. WO9745147A1 describes a method for obtaining porous calcium polyphosphate for its use in the regeneration of the interface between bone and other connecting tissue. The method again synthesizes a porous calcium polyphosphate structure by submitting monocalcium phosphate to several heating steps. Hydrochloric acid is used to dissolve part of the calcium polyphosphate and to contribute to the formation of porosity. The calcium polyphosphate obtained by the method presents an exclusively beta crystalline form.

In another example, patent no. US7494614 describes a method for producing a porous beta calcium polyphosphate structure that also includes several heating steps. Monocalcium phosphate (MCP) is processed in order to produce amorphous calcium polyphosphate at a range then being selected. The selected granules are then inserted into a mould. The contents of the mould are then heated at various temperatures until the crystallisation of the amorphous calcium polyphosphate takes place.

In a further example, patent application no. WO03055418A1 describes the production of porous structures of several calcium phosphates by performing several heating steps on an initial material. In addition, organic and inorganic acids such as hydrochloric acid are used as catalysts to dissolve part of the material and aid the formation of a porous structure.

WO03/055418 discloses the preparation of a porous calcium polyphosphate bone replacement material prepared by sintering monocalcium phosphate hydrate opt. in admixture with an acid catalyst. As technology evolves, biomaterials used in bone regeneration are being forced to comply with new requirements. For instance, it is becoming increasingly desirable that once new biomaterials are placed in contact with a platelet-rich formulation, biomaterials are capable of encouraging the activation of the platelets in said formulation so that the growth-factor content is released from the platelets and fibrin is formed (the activation of platelets and the formation of fibrin are necessary to encourage the regeneration of tissue). Obviously, not all biomaterials have this ability. For example, a recent study by Cho HS, Park SY, Kim S, et al. entitled "Effect of different bone substitutes on the concentration of growth factors in platelet-rich plasma" (J Biomed Appl 2008;22:545-557), analyses the ability of two ceramics very widely used as biomaterials in bone regeneration (hydroxyapatite and calcium polyphosphate) to activate platelets. The results of the study indicate that none of these materials is capable of activating the platelets.

It is an objective of the present invention to propose a new method for obtaining a porous calcium polyphosphate structure that solves at least one of the preceding problems.

In other words, the method must be easy to carry out and it must comprise fewer steps than the methods known in the prior art.

In addition the method must, in at least some of its embodiments, produce a new biomaterial that, when in contact with a platelet-rich formulation, has the ability to activate platelets contained in said formulation in order to release its content of growth factors and induce the formation of fibrin.

The biomaterial obtained by method according to the invention can be suitable for use in bone regeneration and in other applications in different fields of medicine.

### Brief description of the invention

In order to overcome one or more of the aforementioned problems, a method is proposed for producing a porous calcium polyphosphate structure, the method comprising the steps of mixing monocalcium phosphate (MCP) with silicic acid and of sintering the mixture at a predetermined temperature or temperatures for a predetermined time, thus obtaining a porous calcium polyphosphate. Sintering is understood as having the mixture heated at a temperature below fusion temperature of the mixture.

The advantage of starting with a mixture of monocalcium phosphate (MCP) with silicic acid, instead of starting with unmixed MCP as described in prior art, is that it is possible to achieve biomaterials of a different porosity and a different crystalline phase (beta or beta+gamma) by varying the ratio between the silicic acid and MCP and by varying the sintering temperature. In addition, the resulting biomaterial has the ability to activate the platelets of a possible platelet-rich compound in contact with the biomaterial, thanks to the presence of silicon ions in the biomaterial. The method according to the invention is also easy to carry out.

### Brief description of the drawings

Details of the invention can be seen in the accompanying nonlimiting drawings:
- FIG. 1 shows different photographs of biomaterials obtained by carrying out the method of the invention using different mixture ratios of MCP and silicic acid.
- FIG. 2 shows a photograph of a melted material obtained after sintering at 1000°C.
- FIG. 3 shows a photograph of a biomaterial obtained through preheating at 75°C and of a non-inflated material obtained through preheating at 230°C.
- FIG. 4 shows photographs of biomaterials obtained as a result of having added of different quantities of calcium carbonate.
- FIG. 5 shows electronic microscope images of a calcium polyphosphate synthesised from MCP only and of a calcium polyphosphate synthesised from MCP mixed with silicic acid modified with calcium carbonate at 10%.
- FIG. 6 shows the X-ray diffraction pattern of a ceramic prepared according to an embodiment of the inventive method.
- FIG. 7 shows the X-ray diffraction pattern of a ceramic prepared according to another embodiment of the inventive method.
- FIG. 8 shows the formation of a fibrin membrane that agglutinates the ceramic prepared according to the invention.
- FIG. 9 shows a graph comparing cell proliferation in a composite of plasma rich in growth factors and a calcium polyphosphate obtained from MCP only, with cell proliferation in a composite of plasma rich in growth factors and a calcium polyphosphate obtained according to the invention.

### Detailed description of the invention

A method is defined for producing a porous calcium polyphosphate structure, which comprises the steps of:
a) Mixing monocalcium phosphate (MCP) with silicic acid at a weight/volume ratio between 1 and 50 g/ml.
b) Sintering the mixture at a temperature below 980ºC for a predefined time, with the result that the mixture inflates and a porous calcium polyphosphate is obtained. Sintering is understood as heating the mixture at a temperature below the fusion temperature of the mixture. When the MCP is heated, the mass inflates and the porous calcium polyphosphate structure is created.

The monocalcium phosphate (MCP) used in step (a) is preferably monocalcium phosphate monohydrate. Using said specific formulation allows to maximize the porosity of the final porous structure obtained after step (b).

The method thus combines the production of a porous structure and the formation of the calcium polyphosphate in a single step (the sintering step), resulting in a very simplified method that, in comparison with the prior art, significantly reduces the number of steps needed to generate the porous structure. For example, in the method described in Pilliar RM, et al. Biomaterials 2001;22:963-973, five steps are carried out in creating the porous calcium polyphosphate structure: a first step of heating MCP; a second step of melting the MCP; a third step of cooling the material very quickly; a fourth step of selecting the suitable granules; and a fifth step of, again, heating. In the method described in patent US7494614 various heating phases are also carried out.

The method according to the invention allows calcium polyphosphate biomaterials of varying porosity to be obtained, depending on the ratio of the mixture of MCP and silicic acid, and on to the sintering temperature. As a result, biomaterials having a controlled and therefore stable porosity may be obtained. Because the porosity of a biomaterial directly influences its degradation and its mechanical properties (see, for example, Wang Q, Wang Q, Wan C. The effect of porosity on the structure and properties of calcium polyphosphate bioceramics. Ceramics-Silikaty 2011;55:43-48, which describes how the dissolution and compressive strength of a biomaterial respectively rises and decreases when its porosity is increased), being able to control the porosity of the final biomaterial allows the invention to obtain non-easily-breakable biomaterials. In addition, porosity is an important factor that regulates the release of bioactive materials and medicines from a matrix. The increase in porosity speeds up the release of these bioactive materials from the ceramics and calcium cements [Alkhraisat MH, Rueda C, Cabrejo-Azama J, et al. Loading and release of doxycycline hyclate from strontium-substituted calcium phosphate cement. Acta Biomater 2010;6:1522-1528].

Moreover, adjusting of the ratio of the mixture of MCP and silicic acid and/or adjusting of the sintering temperature allows not only to adjust the total porosity but also to control pore distribution according to size (macro-, meso-, and micro-pores). Each size of pore may be of interest for different reasons and depending on the application. For example, the presence of macropores (≥ 100 µm) and mesopores (10-100 µm) influences the *in vivo* degradation of the biomaterial, and also allows cells to penetrate the porous structure and vascular growth to take place, which guarantees blood flow in the new tissue formed in the porous structure. It is also important that the porous structure has a population of micropores (< 10 µm) as this guarantees interconnectivity between the pores and increases the specific surface area [Wei J et al. Hierarchically microporous/macroporous scaffold of magnesium-calcium phosphate for bone tissue regeneration; Biomaterials 2010;31:1260-1269]. This interconnectivity also guarantees the diffusion of nutrients and of secondary products of cellular metabolism.

The table shown below presents the porosities of different biomaterials, which have been obtained in the following ways: by sintering MCP only (as known in prior art); and by sintering different mixtures of MCP and silicic acid at different temperatures and in different concentrations, with the mixtures containing different proportions of MCP (powder) and silicic acid (liquid). Porosities were measured by a measuring method involving high-pressure mercury porosimetry.

**Table 1. Porosity of calcium polyphosphate prepared from MCP modified with silicic acid.**

| **Material** | **Powder/liquid ratio** | **Porosity** | **Macropores** | **Mesopores** | **Micropores** |
|---|---|---|---|---|---|
| Sintered at 500ºC for ten hours | | | | | |
| MCP only (prior art) | - | 42.09% | 55.70% | 47% | 8.70% |
| MCP + silicic acid 38.3% | 7.5 g/ml | 43.40% | 56.90% | 26.04% | 17.06% |
| MCP + silicic acid 75.6% | 7.5 g/ml | 49.01% | 55.20% | 35.20% | 20% |
| MCP + silicic acid 75.6% | 30 g/ml | 63.97% | 61.60% | 25.70% | 12.70% |

| Sintered at 650ºC for ten hours | | | | | |
|---|---|---|---|---|---|
| MCP only | - | 56.19% | 73.40% | 19.46% | 7.40% |
| MCP + silicic acid 75.6% | 7.5 g/ml | 50.46% | 68.67% | 16.50% | 14.83% |
| MCP + silicic acid 86.1% | 7.5 g/ml | 54.30% | 60.47% | 28.79% | 10.74% |

| Sintered at 75ºC for five hours and at 650ºC for ten hours | | | | | |
|---|---|---|---|---|---|
| MCP + silicic acid 75.6% | 4.5 g/ml | 60.83% | 60.71% | 30.69% | 8.60% |

| Sintered at 750ºC for ten hours | | | | | |
|---|---|---|---|---|---|
| MCP + silicic acid 75.6% | 7.5 g/ml | 48.62% | 64.28% | 22.36% | 13.36% |

As can be observed in the table above, the method according to the invention not produces greater porosity in comparison to the biomaterial obtained from MCP only, but also obtains a greater population of micropores and this improves pore size distribution. It should be remembered that increasing the micropore population improves interconnectivity between the macro- and mesopores. In turn, the population of macropores remains stable or increases in comparison with the biomaterial prepared from MCP only.

MCP is mixed with silicic acid at a weight/volume ratio of between 1 and 50 g/ml, as it is in this range that the variations in properties yielding the best results are caused.

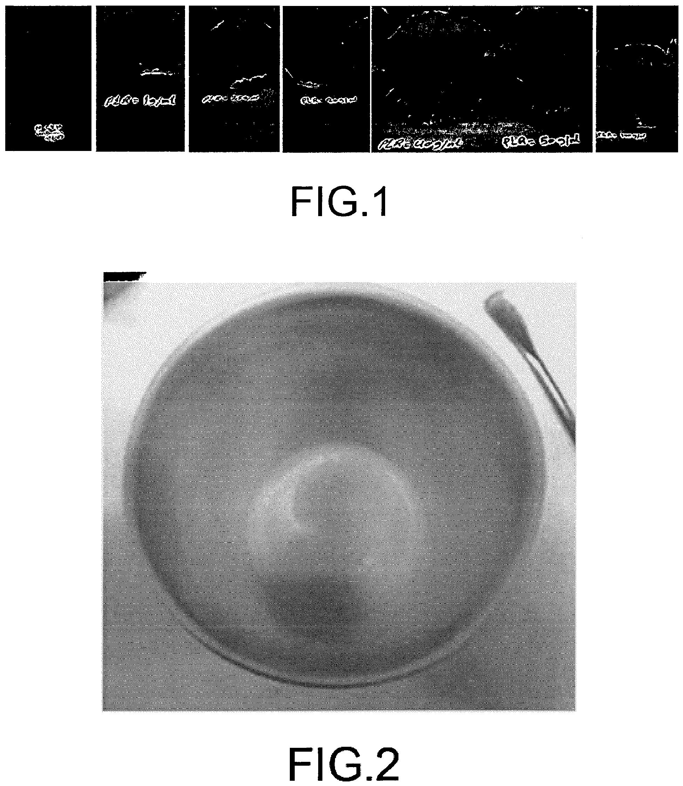

For example, in a practical case different mixtures of monocalcium phosphate and 75.6% (v/v) silicic acid solution were prepared at different mixture ratios of 1, 4.5, 7.5, 20, 40, 50 and 100 g/ml (grams of MCP powder per millilitres of liquid silicic acid). The mixtures were heated at a temperature of 75ºC for five hours, and then at a temperature of 650ºC for ten hours. A calcium polyphosphate produced from MCP only and following the same protocol was used as a benchmark. Different biomaterials were obtained, shown in the photographs in FIG. 1, and their porosities were compared. The results indicate that the porous structure produced according to this protocol was similar in the case of the calcium polyphosphate produced at a powder/liquid ratio of 40, 50 and 100 g/ml (the last three photographs in the figure) and in the case of the benchmark biomaterial (the first photograph in the figure), with the form of these structures not being excessively strong and breaking quite easily. In contrast, the powder/liquid ratios of 4.5 (not photographed), 7.5 and 20 g/ml (the third and fourth photograph respectively) were capable of producing stronger porous structures and in a more stable manner. Finally, the powder/liquid ratio of 1 g/ml resulted in a dense structure, which is shown in the second photograph.

In another example, different mixtures of monocalcium phosphate with 75.6% (v/v) silicic acid solution were carried out at different mixture ratios of 1, 20, 40, and 50 g/ml. The calcium polyphosphate was produced by heating the mixture at a temperature of 75ºC for five hours, and then at a temperature of 650ºC for ten hours. Following sintering, the ceramics were ground, with a granule size of between 0.5 and 0.8 mm being selected. 0.15 g of the granules of each material were incubated in a phosphate buffer solution (PBS; pH=7.3) for 24 hours. The pH test revealed pH values of 2 and between 6 and 7 for the samples prepared respectively with a powder/liquid ratio of 1 and 20 g/ml. The pH of the PBS did not change in the case of the samples prepared with a powder/liquid ratio of 40 and 50 g/ml. This shows that the solubility (degradability) of the calcium polyphosphate may be varied by selecting the value of the powder/liquid ratio (mass of MCP by volume of silicic acid).

As regards the step in which the mixture is sintered for the purpose of obtaining a porous calcium polyphosphate structure, as mentioned heretofore, said sintering is carried out at a temperature below 980ºC. This allows porous calcium polyphosphate to form successfully in the crystalline structure of the biomaterial. Sintering the calcium polyphosphate at a temperature greater than or equal to 980ºC would in contrast cause the fusion of the calcium polyphosphate (Wang K. The effect of a polymericchain-like structure on the degradation and cellular biocompatibility of calcium polyphosphate. Materials Science and Engineering C 2008;28:1572-1578) and also the production of amorphous calcium polyphosphate, neither of these effects being of interest to the present invention.

In a practical example 15 g of MCP were mixed with 2 ml of a silicic acid solution having a silicon ion concentration of 86.1% (v/v)). The mixture was sintered at a temperature of 1000ºC for ten hours, causing the fusion of the calcium polyphosphate and preventing the formation of a porous structure. FIG. 2 shows a photograph of the melted material obtained.

In an especially advantageous manner, sintering is carried out at a temperature of between 500 and 750ºC. Although a porous calcium polyphosphate structure is obtained at a temperature of 500ºC, increasing the temperature to values of 650ºC and 750ºC hardens the porous structure further and allows the porosity to be adjusted. In addition, in order to grind the calcium polyphosphates into granules (which is useful for selecting a suitable size of granule for filling the bone defects and encouraging the formation of new bone; for facilitating its use to increase the volume of autologous bone graft obtained during surgery; for facilitating its mixture with liquids such as plasma rich in platelets or blood; and for facilitating its application in bone defects of different shapes and sizes), it has been observed that more force is needed to break the calcium polyphosphate produced at temperatures of 650ºC and 750ºC than the calcium polyphosphate synthesised at 500ºC.

For example, a mixture of MCP and 75.6% silicic acid was sintered at a ratio of 7.5 g/ml at three different temperatures (500, 650 and 750ºC) for ten hours. The results, shown in the table below (which is an extract of Table 1), reflect that increasing the temperature allowed for obtaining structures with a different number of macropores, mesopores and micropores and with a variable total porosity.

**Table 2. Porosity of calcium polyphosphate prepared from MCP modified with silicic acid at 75.6% at 7.5 g/ml and at different temperatures.**

| **Material** | **Powder/liquid ratio** | **Porosity** | **Macropores** | **Mesopores** | **Micropores** |
|---|---|---|---|---|---|
| Sintered at 500ºC for ten hours | | | | | |
| MCP + silicic acid 75.6% | 7.5 g/ml | 49.01% | 55.20% | 35.20% | 20% |

| Sintered at 650ºC for ten hours | | | | | |
|---|---|---|---|---|---|
| MCP + silicic acid 75.6% | 7.5 g/ml | 50.46% | 68.67% | 16.50% | 14.83% |

| Sintered at 750ºC for ten hours | | | | | |
|---|---|---|---|---|---|
| MCP + silicic acid 75.6% | 7.5 g/ml | 48.62% | 64.28% | 22.36% | 13.36% |

In the event that sintering is carried out a temperature of between 500 and 750ºC, the method according to the invention optionally comprises the step of heating the mixture at a temperature below 200ºC, which is carried out prior to sintering. This prior step maximizes inflation of the monocalcium phosphate mass.

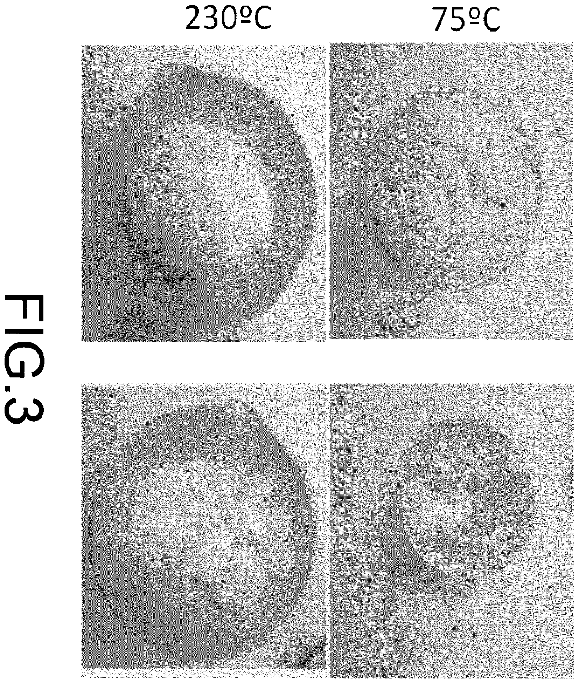

For example, a mixture of MCP and a silicic acid solution with a silicon ion concentration of 75.6% (v/v) was sintered in accordance with two protocols: heating at a temperature of 75ºC for five hours followed by a temperature of 650ºC for ten hours; and heating at a temperature of 230ºC for five hours followed by a temperature of 650ºC for ten hours. Results showed that inflation did take place during the first protocol, while no inflation took pace during the second protocol (FIG. 3 shows how the first material was inflated, whereas the second retained its initial consistency).

Preferably, sintering is carried out for a period of time greater than or equal to two hours. This ensures that the mixture is transformed into calcium polyphosphate. In an especially advantageous manner, sintering is carried out for a period of time of between five and ten hours to ensure that sintering does not last for too long and to ensure that a porous structure is obtained rather than a material of another form (for example, a powder). For instance, a mixture of MCP and a silicic acid solution with a silicon ion concentration (v/v) of 76.5% was formed and said mixture was sintered at a temperature of 500ºC for two different time periods of ten hours and 20 hours. Sintering for ten hours resulted in a solid porous structure, whereas sintering for 20 hours resulted in a powder.

Optionally, during the mixing step, the MCP and the silicic acid are also mixed with a source of calcium ions. The source of calcium ions is preferably calcium carbonate and/or calcium hydroxide, as these compounds do not contribute other additional ions that may not be suitable for the biomaterial. The calcium ions contribute to increase porosity, as can be observed in the table below, which shows the porosity of the biomaterials resulting from sintering the following starting materials at 650ºC: MCP only (prior art); MCP mixed with 75.6% silicic acid at a ratio of 7.5 g/ml; MCP mixed with 86.1% silicic acid at a ratio of 7.5 g/ml; and the two aforementioned materials mixed also with calcium hydroxide, which acts as a source of calcium ions.

**Table 3. Porosity of calcium polyphosphate prepared from MCP modified with silicic acid, optionally with calcium hydroxide.**

| Material | Powder/liquid ratio | Poro-sity | Macropores | Mesopores | Micropores |
|---|---|---|---|---|---|
| 650ºC for ten hours | | | | | |
| MCP only | - | 56.19% | 73.40% | 19.46% | 7.40% |
| MCP + silicic acid 75.6% | 7.5 g/ml | 50.46% | 68.67% | 16.50% | 14.83% |
| MCP + silicic acid 86.1% | 7.5 g/ml | 54.30% | 60.47% | 28.79% | 10.74% |
| MCP + silicic acid 86.1% | 7.5 g/ml + 5%Ca(OH)2 | 68.05% | 69.17% | 24.04% | 6.79% |
| MCP + silicic acid 86.1% | 7.5 g/ml + 10%Ca(OH)2 | 72.81% | 53.09% | 34.81% | 12.1% |

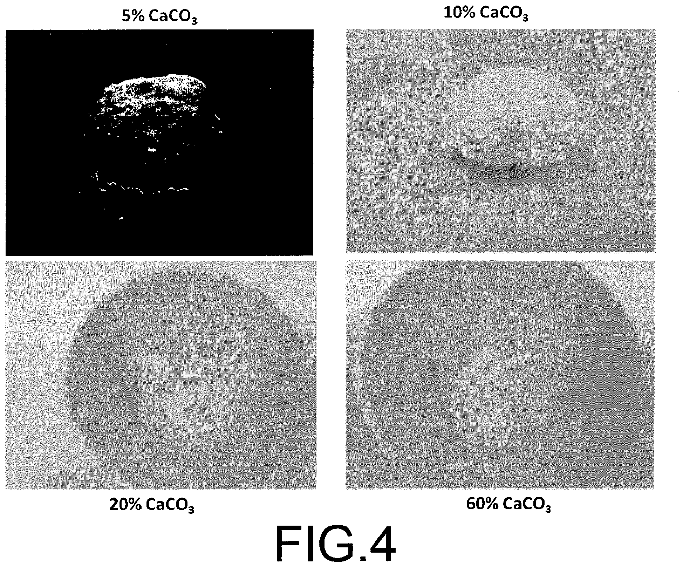

With regard to the use of calcium carbonate (CaCO₃), in an example calcium carbonate was added to the mixture of MCP and a 75.6% (v/v) silicic acid solution (in a powder/liquid ratio of 7.5 g/ml) at a ratio (weight/weight) of 5%, 10%, 20%, and 60%. The results indicated that the porosity of the calcium polyphosphate increased at concentrations lower than 20%, whereas concentrations equal to or greater than 20% compacted the calcium polyphosphate and produced poorly cohesive structures that became reduced to powder, as shown in FIG. 4.

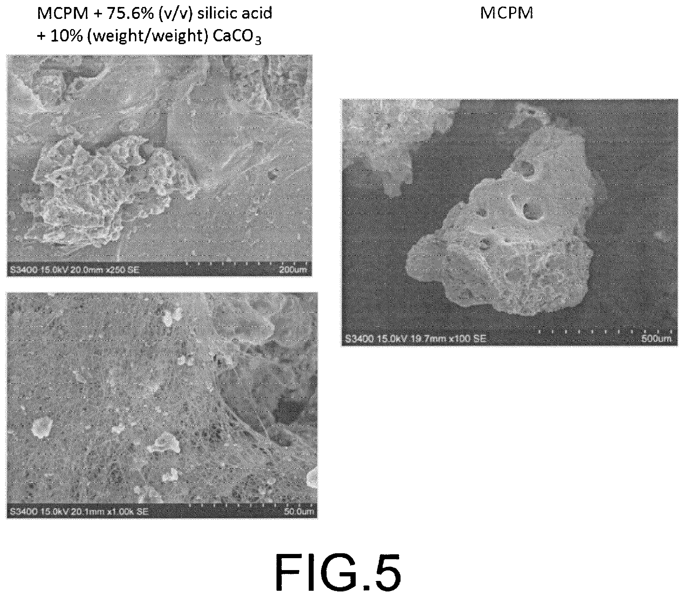

In a further example, MCP alone was sintered at 500ºC for ten hours, resulting in a calcium polyphosphate compound that when incubated in water reduced its pH to a value of around 2. The use of calcium carbonate improved this aspect by softening the reduction in the pH of the water until an alkaline pH was obtained at CaCO₃ concentrations in excess of 20%. This modification has contributed to improving the stability of fibrin in a culture medium. For example, a growth-factor-rich plasma was mixed with two calcium polyphosphates, respectively synthesised from MCP and from MCP mixed with silicic acid and modified with 10% calcium carbonate. Following seven days' incubation in a culture medium, visual checks and inspection with a scanning electron microscope confirmed the presence of fibrin only in the calcium polyphosphate modified with calcium carbonate. FIG. 5 shows the stability of fibrin formed in two calcium polyphosphates: a calcium polyphosphate prepared using MCP only; and calcium polyphosphate prepared using MCP mixed with 75.6% (v/v) silicic acid and modified with 10% (weight/weight) calcium carbonate. The samples were incubated in a culture medium for seven days.

The invention also contemplates the possibility of adding calcium carbonate and calcium hydroxide jointly. In relation to this, tests have been performed in which both compounds have been added jointly, with the concentration of each of them varying between 10 and 40%. As a result, compact structures have been obtained. These structures were low in consistency and were reduced to granules when handled. The incubation of these granules in water gave rise to an alkaline pH.

Optionally, the method according to the invention comprises the step of adding a source of calcium ions after the sintering phase. In an example, calcium carbonate and calcium hydroxide were added separately and in a concentration of 40% to a previously-synthesised calcium polyphosphate, where sintering had been carried out at 650ºC from a mixture of MCP and a 75.6% (v/v) silicic acid solution (at a powder/liquid ratio of 7.5 g/ml). As a result, the pH of the water was alkaline after the sample was incubated. Granules, rather than structures, were obtained.

Optionally, the method according to the invention comprises the previous step of obtaining silicic acid by hydrolysing a source of silicon ions in an aqueous acid solution. The main objective of using the aqueous solution of silicon ions is not to load the MCP with silicon ions but to produce structures of varying degrees of porosity (amount of pores and pore size distribution). A polycrystalline structure is obtained by heating at only one temperature in the range of temperatures between 500ºC and 980ºC.

Preferably, the relation between the volume of the source of silicon ions and the total volume of the solution is between 10 and 90%. The variation in the concentration of this source of silicon is also an effective parameter for controlling the porosity of the calcium polyphosphate. In an example, a silicic acid solution can be prepared by hydrolysising tetraethyl orthosilicate (TEOS) using a solution of hydrochloric acid with a pH equal to 2. 0.1, 1.99, 3.83, 5.5, 6.08, and 7.56 ml of TEOS are mixed (by magnetic stirring) with 9.9, 8.01, 6.17, 4.5, 3.92, 2.44, and 1.39 ml of 0.01M HCl until a clear solution is obtained. The mixtures are then stored at 4ºC overnight to complete the hydrolysis of the TEOS. Solutions with silicon-ion concentrations of 1%, 19.9%, 38.3%, 55%, 60.8%, 75.6%, 86.1% are obtained as a result. These solutions may be used to produce the biomaterial described in this invention, as shown in the examples described in this document.

An additional effect of the method according to the invention is that porous calcium polyphosphate structures having different crystalline structures (beta phase and/or gamma phase) are able to be obtained from MCP. In conventional methods starting with MCP alone, it is only possible to obtain one crystalline structure. Or, alternatively, conventional methods must start with calcium polyphosphate in order to achieve the coexistence of the beta and gamma phases [Guo L. et al.; Phase transformations and structure characterization of calcium polyphosphate during sintering process; Journal of Materials Science 39 (2004) 7041-7047]. Each crystalline structure or combination of crystalline structures presents different properties and may therefore be of interest in different applications. For example, the gamma phase of calcium polyphosphate has been described as being more soluble than its beta phase [Jackson LE, et al. Key Engineering Materials 2008;361-363:11-14]. The coexistence of the beta and gamma phases can therefore affect the biomaterial's solubility and thus allow controlling its *in vivo* reabsorption. In addition, from a scientific point of view it may be interesting to study the physical and chemical properties of the gamma phase and its combination with the beta phase.

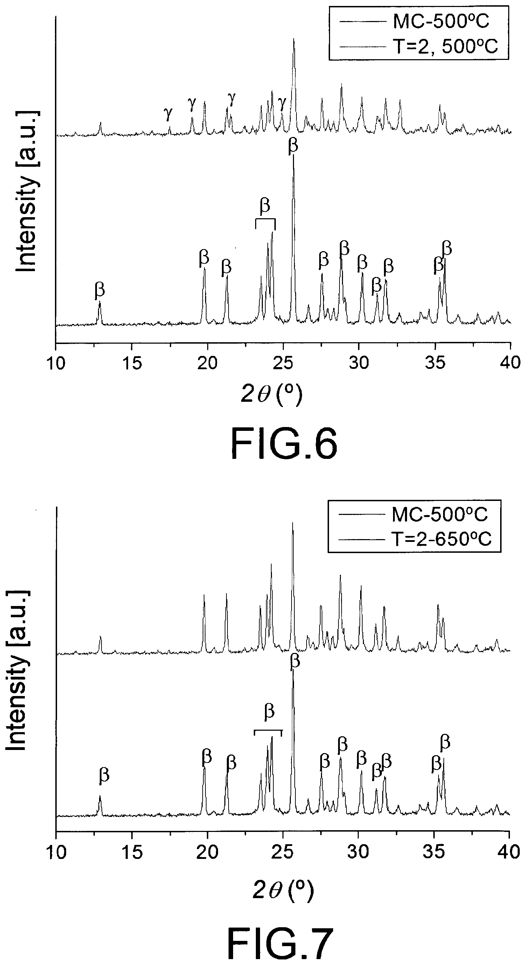

In an example, 15 g of MCP were mixed with 2 ml of a solution containing Si-OH prepared using TEOS with a silicon ion concentration of 86.1% (v/v). The mixture was sintered at a temperature of 500ºC for ten hours. The X-ray diffraction showed that the resulting ceramic comprised the beta and gamma forms of calcium polyphosphate, as can be observed in the graph in FIG. 6. Said graph shows the X-ray diffraction pattern of the ceramic, where the symbol *β* indicates the diffraction peaks that correspond to the beta form and the symbol *γ* indicates the peaks of the gamma form.

In another example, 15 g of MCP were mixed with 2 ml of a solution containing Si-OH prepared using TEOS with a silicon ion concentration of 86.1% (v/v). The mixture was sintered at a temperature of 650ºC for ten hours. The X-ray diffraction showed that the resulting ceramic only comprised the beta form, as can be seen in the graph in FIG. 7. Said graph shows the X-ray diffraction pattern of the ceramic, where the symbol *β* indicates the diffraction peaks that correspond to the beta form and the symbol *γ* indicates the peaks of the gamma form.

Optionally, the method according to the invention comprises the previous step of adding biologically effective ions such as magnesium, zinc, strontium, sodium, potassium, copper and iron ions to the MCP and/or the silicic acid. Alternatively or additionally, the method may comprise the step of mixing the porous structure obtained after the sintering with solutions or liquids that contain said ions. The purpose of these two options is to allow said ions with biological effects to be incorporated into the material's structure, so that as the biomaterial degrades the ions are released in the area where the biomaterial is situated.

In summary, mixing monocalcium phosphate (MCP) with silicic acid allows for obtaining a stable and tough porous biomaterial, the porosity of which may be varied depending on specific parameters of the method. The parameters of the method that may be adjusted in order to regulate and control the porosity and the crystalline phases of the biomaterial are: the silicic acid concentration; the mixing ratio of MCP and silicic acid; the sintering temperature; the duration of the sintering; a possible preheating; and/or the possible addition of calcium ions.

An additional advantage of the method according to the invention is that it allows valid porous structures to be produced in conditions in which they could not be produced if only monocalcium phosphate were being used as a starting material. For example, in a practical case the starting material was MCP only and it was subjected to a temperature below 200ºC for at least one second, followed by sintering at a temperature below 980ºC. A fragile porous structure incapable of maintaining its form was obtained. In contrast, if the starting material is MCP mixed with silicic acid, as has been shown in the examples in this document, a stable porous biomaterial is obtained, whose porosity varies depending on the exact silicon concentration and powder/liquid ratio conditions.

Another advantage of the method according to the invention is that it allows producing porous structures formed similarly to bone. More specifically, the porous structures obtained by the method present a denser outer layer and a more porous inner layer, similar to the very dense cortical bone layer and the more porous inner bone tissue.

A further advantage of the method according to the invention is that the porous calcium polyphosphate structure obtained by the method has the ability to activate platelets contained in a platelet-rich formulation. Thus, the structure can optimally assist platelet-rich formulations designed for tissue regeneration in performing their regenerative function.

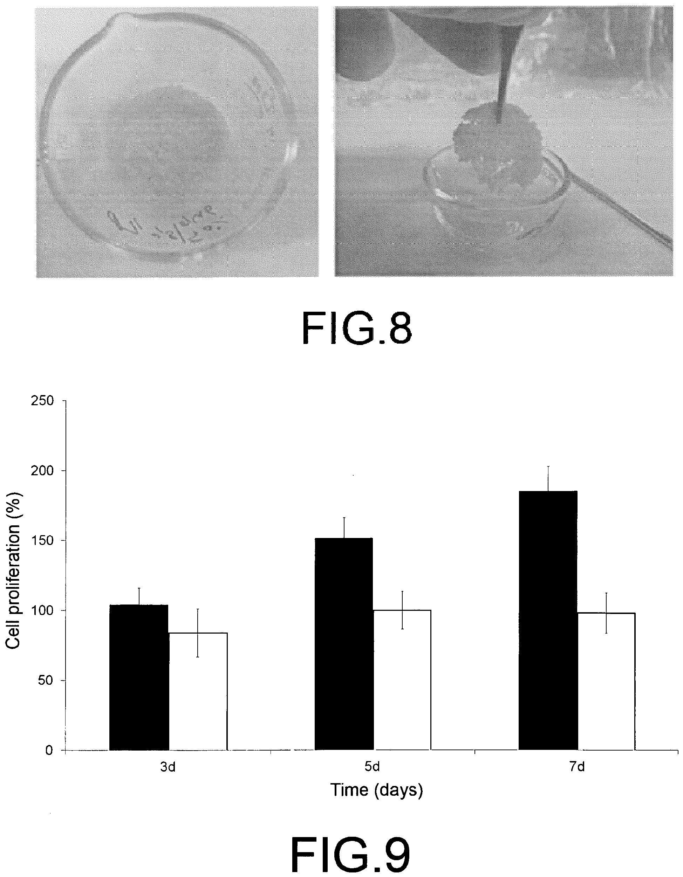

For example, 0.3 g of the porous biomaterial (prepared with a silicic acid solution at 86.1% and by sintering at 650ºC for ten hours) were mixed with 600 µl of the fraction richest in platelets (and therefore in growth factors) of a blood plasma centrifuged in accordance with the method described in patent US6569204B1. The mixture was incubated at 37ºC for 10 minutes. FIG. 8 shows that a fibrin membrane agglutinating the particles of the ceramic was formed, which indicates that activation of platelets contained in the fraction of plasma did take place, i.e. growth factors were released from the platelets.

In another example, 0.02 g of the porous biomaterial (prepared with a silicic acid concentration at 75.6% and 10% CaCO₃) was mixed with 500 µl of a blood plasma centrifuged in accordance with the method described in patent US6569204B1. After between 30 and 40 minutes at ambient temperature the fibrin formed and retracted. The supernatant liquid was collected so that its growth-factor content could be analysed. The results indicated that the concentrations of the platelet-derived growth factor (PDGF-AB) and the beta transforming growth factor (TGF-β) were 10,039.59 pg/ml ± 368.28 and 42,700 pg/ml ± 2,121 respectively. In other words, the presence of the porous biomaterial prepared in accordance with the invention caused the release of the growth factors contained in the platelets present in the plasma (i.e. caused the activation of the platelets), thereby demonstrating the potential of the biomaterial prepared according to this invention to activate the platelets and to induce the formation of fibrin.

A further advantage of the method according to the invention is that the biomaterial obtained by the method can have a significant ability to promote cell growth, and thus be used as a culture medium. In this regard, for instance, the ability of two composites formed by a porous calcium polyphosphate and a growth-factor-rich plasma to promote cell growth was tested. One of the composites comprised calcium polyphosphate synthesised from MCP alone and the other composite comprised calcium polyphosphate synthetised from MCP and silicic acid at 75.6% (PLR = 7.5 g/ml), in accordance with the invention. Cell cultures in a culture medium without fetal bovine serum were developed. The results, shown in FIG. 9, clearly indicate that the calcium polyphosphate of the mixture modified with silicic acid (shown in black) improved the proliferation of MG63 osteoblast-like cells significantly more than the calcium polyphosphate synthesised from MCP only (shown in white).

Optionally, during sintering the mixture is compacted to provide the material with a certain shape. The porous biomaterial obtained by the method is a calcium phosphate that presents a porosity greater than or equal to 30%, preferably between 40 and 80%, with a population of macropores greater than or equal to 40%, preferably between 50 and 75%; a population of mesopores greater than or equal to 10%, preferably between 10-50%; and a population of micropores greater than or equal to 4%, preferably between 5 and 30%.

It is another object of the invention to use the biomaterial obtained according to the inventive method of manufacturing as a filling material, designed to fill a cavity generated by a defect in a bone tissue having been produced.

It is another object of the invention to use the biomaterial obtained according to the inventive method as a support medium for cell growth, in other words, as a medium for allowing the cells (e.g. osteoblasts) to proliferate on the surface of the material.

It is another object of the invention to use the biomaterial obtained according to the inventive method to reinforce organic matrices such as polymers, or gels such as fibrin, hyaluronic acid, hyaluronate salts, chondroitin-4-sulfate, chondroitin-6-sulfate, dextran, silica gel, alginate, hydroxypropyl methylcellulose, chitin derivatives (preferably chitosan), xanthan gum, agarose, polyethylene glycol (PEG), polyhydroxyethyl methacrylate (HEMA), synthetic or natural proteins, collagens or any combination of them.

It is another object of the invention to provide a biomaterial obtained according to the inventive method as a matrix for *in situ* release of medicines, proteins and growth factors. In other words, the biomaterial may be loaded with at least one medicine, protein or growth factor, for release of said medicine, said protein or said growth factor in the area where the biomaterial is located.

## Claims

1. Method for producing a porous calcium polyphosphate structure, **characterised in that** it comprises the steps of:
- mixing monocalcium phosphate (MCP) with silicic acid at a weight/volume ratio between 1 and 50 g/ml; and
- sintering the mixture at a temperature below 980ºC to obtain a porous calcium polyphosphate.

2. Method according to claim 1, wherein sintering is carried out at a temperature of between 500 and 750ºC.

3. Method according to claim 2, further comprising a step in which the mixture is heated at a temperature below 200ºC, said step being carried out prior to the sintering.

4. Method according to claim 1, wherein the sintering is carried out for a period of time greater than or equal to 2 hours.

5. Method according to claim 4, wherein the sintering is carried out for a period of time of between 5 and 10 hours.

6. Method according to claim 1, wherein in the step of mixing monocalcium phosphate (MCP) with silicic acid, a source of calcium ions is also mixed.

7. Method according to claim 1, wherein it comprises the step of adding a source of calcium ions after the sintering phase.

8. Method according to claim 7, wherein the source of calcium ions comprises calcium carbonate.

9. Method according to claim 7, wherein the source of calcium ions comprises calcium hydroxide.

10. Method according to claim 1, further comprising a prior step of obtaining silicic acid by hydrolysing a source of silicon ions in an aqueous acid solution with a ratio between the volume of said source of silicon ions and the total volume of the solution of between 1 and 99%.

11. Method according to claim 10, wherein the ratio between the volume of the source of silicon ions and the total volume of the solution is between 10 and 90%.

12. Method according to claim 1, wherein during sintering the mixture is compacted to provide the material with a certain shape.

13. Method according to claim 1, further comprising a prior step in which ions with biological effects are added to the MCP and/or the silicic acid.

14. Method according to claim 1, comprising a step in which the porous structure obtained after sintering is mixed with solutions or liquids containing ions with biological effects.

15. Method according to claim 1, wherein the monocalcium phosphate is monocalcium phosphate monohydrate.

16. A calcium polyphosphate prepared in accordance with the method of claim 1 and presenting a porosity greater than or equal to 30%, preferably between 40 and 80%, with a population of macropores greater than or equal to 40%, preferably between 50 and 75%; a population of mesopores greater than or equal to 10%, preferably between 10 and 50%; and a population of micropores greater than or equal to 4%, preferably between 5 and 30%.

17. A material for filling bone cavities, which comprises a porous calcium polyphosphate structure manufactured according to the method of claim 1.

18. A medium for cell growth, which comprises a porous calcium polyphosphate structure manufactured according to the method of claim 1.

19. A material for reinforcing organic matrices, which comprises a porous calcium polyphosphate structure manufactured according to the method of claim 1.

20. A matrix material for being loaded with a medicament, a protein or a growth factor and for allowing the same to become released on said matrix material, **characterized in that** it comprises a porous calcium polyphosphate structure manufactured according to the method of claim 1.

## Patentansprüche

1. Methode zur Erzeugung einer porösen Kalziumpolyphosphat-Struktur, **dadurch gekennzeichnet, dass** sie folgende Schritte umfasst:
- die Mischung von Monokalziumphosphat (MCP) mit Kieselsäure in einem Gewicht/Volumenverhältnis zwischen 1 und 50 g/ml; und
- Sinterung der Mischung bei einer Temperatur von unter 980°C, um ein poröses Kalziumpolyphosphat zu erhalten.

2. Methode gemäß Anspruch 1, wobei die Sinterung bei einer Temperatur zwischen 500 und 750°C durchgeführt wird.

3. Methode gemäß Anspruch 2, die ferner einen Schritt umfasst, bei dem die Mischung bei einer Temperatur unter 200°C erhitzt wird, wobei besagter Schritt vor der Sinterung durchgeführt wird.

4. Methode gemäß Anspruch 1, wobei die Sinterung für einen Zeitraum von mehr oder gleich 2 Stunden durchgeführt wird.

5. Methode gemäß Anspruch 4, wobei die Sinterung für einen Zeitraum zwischen 5 und 10 Stunden durchgeführt wird.

6. Methode gemäß Anspruch 1, wobei bei dem Schritt des Mischens von Monokalziumphosphat (MCP) mit Kieselsäure auch eine Kalziumionenquelle gemischt wird.

7. Methode gemäß Anspruch 1, wobei dies den Schritt der Hinzugabe einer Kalziumionenquelle nach der Sinterungsphase umfasst.

8. Methode gemäß Anspruch 7, wobei die Kalziumionenquelle Kalziumkarbonat umfasst.

9. Methode gemäß Anspruch 7, wobei die Kalziumionenquelle Kalziumhydroxyd umfasst.

10. Methode gemäß Anspruch 1, die ferner einen vorangehenden Schritt umfasst, bei dem Kieselsäure durch Hydrolysieren einer Siliziumionenquelle in einer wässrigen Säurelösung mit einem Verhältnis zwischen dem Volumen besagter Siliziumionenquelle und dem Gesamtvolumen der Lösung zwischen 1 und 99% gewonnen wird.

11. Methode gemäß Anspruch 10, wobei das Verhältnis zwischen dem Volumen der Siliziumionenquelle und dem Gesamtvolumen der Lösung zwischen 10 und 90% liegt.

12. Methode gemäß Anspruch 1, wobei während der Sinterung die Mischung verdichtet wird, um dem Material eine bestimmte Form zu verleihen.

13. Methode gemäß Anspruch 1, die ferner einen vorausgehenden Schritt umfasst, bei dem dem MCP und/oder der Kieselsäure Ionen mit biologischen Wirkungen hinzugegeben werden.

14. Methode gemäß Anspruch 1, die einen Schritt umfasst, bei dem die nach der Sinterung erhaltene poröse Struktur mit Lösungen oder Flüssigkeiten gemischt wird, die Ionen mit biologischen Wirkungen enthalten.

15. Methode gemäß Anspruch 1, wobei das Monokalziumphosphat Monokalziumphosphatmonohydrat ist.

16. Ein Kalziumpolyphosphat, das in Übereinstimmung mit der Methode von Anspruch 1 präpariert ist und eine Porosität von größer oder gleich 30% aufweist, vorzugsweise zwischen 40 und 80%, mit einer Population von Makroporen von größer oder gleich 40%, vorzugsweise zwischen 50 und 75%, mit einer Population von Mesoporen von größer oder gleich 10%, vorzugsweise zwischen 10 und 50%, und einer Population von Mikroporen von größer oder gleich 4%, vorzugsweise zwischen 5 und 30%.

17. Ein Material zum Auffüllen von Knochenhohlräumen, das eine poröse Kalziumpolyphosphat-Struktur umfasst, die gemäß der Methode von Anspruch 1 hergestellt ist.

18. Ein Medium für das Zellwachstum, das eine poröse Kalziumpolyphosphat-Struktur umfasst, die gemäß der Methode von Anspruch 1 hergestellt ist.

19. Ein Material zur Verstärkung von organischen Matrizen, das eine poröse Kalziumpolyphosphat Struktur umfasst, die gemäß der Methode von Anspruch 1 hergestellt ist.

20. Ein Matrixmaterial zur Bestückung mit einem Medikament, einem Protein oder einem Wachstumsfaktor, damit dieses/dieser auf besagtem Matrixmaterial freigesetzt wird, **dadurch gekennzeichnet, dass** es eine poröse Kalziumpolyphosphat-Struktur umfasst, die gemäß der Methode von Anspruch 1 hergestellt ist.

## Revendications

1. Méthode pour produire une structure poreuse en polyphosphate de calcium, **caractérisée par le fait qu'**elle comprend les étapes de:
- mélanger du phosphate monocalcique (MCP) avec de l'acide silicique à un ratio poids/volume compris entre 1 et 50 g/ml; et
- fritter du mélange à une température inférieure à 980ºC pour obtenir un polyphosphate de calcium poreux.

2. Méthode selon la revendication 1, où le frittage est réalisé à une température comprise entre 500 et 750ºC.

3. Méthode selon la revendication 2, comprenant par ailleurs une étape dans laquelle le mélange est chauffé à une température inférieure à 200ºC, ladite étape étant réalisée avant le frittage.

4. Méthode selon revendication 1, où le frittage est réalisé pendant une durée supérieure ou égale à 2 heures.

5. Méthode selon la revendication 4, où le frittage est réalisé pendant une durée comprise entre 5 et 10 heures.

6. Méthode selon la revendication 1, où dans l'étape de mélange de phosphate monocalcique (MCP) avec de l'acide silicique est également mélangé une source d'ions de calcium.

7. Méthode selon la revendication 1, qui comprend une étape d'ajout d'une source d'ions de calcium après la phase de frittage.

8. Méthode selon la revendication 7, où la source d'ions de calcium comprend du carbonate de calcium.

9. Méthode selon la revendication 7, où la source d'ions de calcium comprend de l'hydroxyde de calcium.

10. Méthode selon la revendication 1, qui comprend par ailleurs une étape préalable d'obtention d'acide silicique par hydrolyse d'une source d'ions silicium dans une solution acide aqueuse avec un ratio entre le volume de ladite source d'ions silicium et le volume total de la solution compris entre 1 et 99%.

11. Méthode selon la revendication 10, où le ratio entre le volume de la source d'ions silicium et le volume total de la solution est compris entre 10 et 90%.

12. Méthode selon la revendication 1, où pendant le frittage le mélange est compacté pour fournir une certaine forme au matériau.

13. Méthode selon la revendication 1, comprenant également une étape préalable dans laquelle des ions avec des effets biologiques sont ajoutés dans le MCP et/ou l'acide silicique.

14. Méthode selon la revendication 1, comprenant une étape dans laquelle la structure poreuse obtenue après frittage est mélangée avec des solutions ou des liquides contenant des ions avec des effets biologiques.

15. Méthode selon la revendication 1, où le phosphate monocalcique est du phosphate monocalcique monohydrate.

16. Un polyphosphate de calcium préparé selon la méthode de la revendication 1 et présentant une porosité supérieure ou égale à 30%, de préférence entre 40 et 80%, avec une population de macropores supérieure ou égale à 40%, de préférence entre 50 et 75%; une population de mésopores supérieure ou égale à 10%, de préférence entre 10 et 50%; et une population de micropores supérieure ou égale à 4%, de préférence entre 5 et 30%.

17. Un matériau pour remplir les cavités osseuses, qui comprend une structure en polyphosphate de calcium poreuse fabriquée selon la méthode de la revendication 1.

18. Un milieu de croissance de cellules, qui comprend une structure en polyphosphate de calcium poreuse fabriquée selon la méthode de la revendication 1.

19. Un matériau pour renforcer les matrices organiques, qui comprend une structure en polyphosphate de calcium poreuse fabriquée selon la méthode de la revendication 1.

20. Un matériau de matrice à charger avec un médicament, une protéine ou un facteur de croissance pour permettre sa libération sur audit matériau de matrice, **caractérisé par le fait qu'**il comprend une structure en polyphosphate de calcium poreuse fabriquée selon la méthode de la revendication 1.
